# EUROPÄISCHE PATENTANMELDUNG

(11) **EP 2 418 002 A2**
(43) Veröffentlichungstag der Anmeldung: **15.02.2012**
(21) Anmeldenummer: 11154706.3
(22) Anmeldetag: 16.02.2011
(51) Int. Cl.: A61Q 5/00, A61Q 5/12, A61K 8/37, A61K 8/55, A61K 8/64

(54) **Biomimetische Zusammensetzungen zur Pflege und Restrukturierung keratinischer Fasern**

(30) Priorität: 25.06.2010 DE 102010030519
(71) Anmelder: Henkel AG & Co. KGaA, 40589 Düsseldorf (DE)
(72) Erfinder: Schulze zur Wiesche, Erik, 20144, Hamburg (DE); Scheunemann, Volker, 21339, Lüneburg (DE); Freye, Stefanie, 20357, Hamburg (DE)

(57) **Zusammenfassung**

Biomimetische Emulsionen sind hervorragend zur Behandlung keratinischer Fasern geeignet. Gegenstand der vorliegenden Erfindung sind daher kosmetische Zusammensetzungen zur Behandlung keratinischer Fasern, insbesondere menschlicher Haare, enthaltend in einem kosmetischen Träger mindestens ein Öl oder Fett, dessen zugrunde liegende Triglyceride mindestens eine einfach oder mehrfach ungesättigte Fettsäure mit einer Kohlenstoffkettenlänge von mindestens 18 C - Atomen aufweisen, mindestens ein Öl oder Fett, dessen zugrunde Triglyceride mindestens eine gesättigte Fettsäure mit einer Kohlenstoffkettenlänge von mindestens 16 C - Atomen aufweisen, mindestens ein Proteinhydrolysat und weiterhin gegebenenfalls ein Phospholipid.

## Beschreibung

Die kosmetische Behandlung keratinischer Fasern, insbesondere von menschlichen Haaren, ist ein wichtiger Bestandteil der menschlichen Körperpflege. So wird menschliches Haar heute in vielfältiger Weise mit haarkosmetischen Zubereitungen behandelt.

Die bekannten Wirkstoffe können jedoch nicht alle Bedürfnisse in ausreichendem Maße abdecken. Es besteht weiterhin ein Bedarf nach Wirkstoffen bzw. Wirkstoffkombinationen für kosmetische Mittel mit guten pflegenden Eigenschaften und guter biologischer Abbaubarkeit. Weiterhin ist die Verträglichkeit der kosmetischen Zusammensetzungen ein außerordentlich wichtiges Kriterium. Zusammensetzungen zur Anwendung auf keratinischen Fasern, insbesondere menschlichen Haaren müssen nicht nur ein gutes Reinigungs- und Konditionier- sowie Pflegevermögen aufweisen, sondern sollen weiterhin gut verträglich sein und auch bei häufiger Anwendung nicht zu starker Entfettung oder Trockenheit und Spliß führen. Das Anfühlen der keratinischen Fasern nach kosmetischen Behandlungen ist ein wesentliches Kriterium, ob die entsprechende Zusammensetzung vom Verbraucher als angenehm empfunden wird. Die Sensorik und insbesondere die Haptik einer Zusammensetzung sind somit wesentliche Effekte, die der Verbraucher erleben kann. Gesucht sind daher genau solche Zusammensetzungen, welche nicht nur die keratinischen Fasern, insbesondere menschliches Haar, pflegen sondern darüber hinaus durch ihre sensorischen und insbesondere haptischen Eigenschaften der Oberfläche von keratinischen Fasern, insbesondere menschlichen Haaren, spürbar und erlebbar verändern. Darüber hinaus sollen die Zusammensetzungen insbesondere im Inneren der keratinischen Fasern, insbesondere der menschlichen Haare, die Struktur regenerieren und ausgleichen. Dieser Effekt wird auch als Restrukturierung bezeichnet.

Die Aufgabe der vorliegenden Erfindung besteht darin, die konditionierenden Eigenschaften und den Glanz von keratinischen Fasern bei der Anwendung von Zusammensetzungen zur Haarbehandlung deutlich zu verbessern und ihnen darüber hinaus zusätzlich sensorisch, insbesondere haptisch erlebbare Effekte zu verleihen. Dabei sollen die Zusammensetzungen überwiegend auf Inhaltsstoffen beruhen, welche aus natürlichen Rohstoffen gewonnen werden, und welche mit möglichst wenigen chemischen Verfahrensschritten modifiziert wurden und somit weitestgehend den natürlichen Inhaltsstoffen nahe kommen.

In der Vergangenheit wurde immer wieder versucht mit natürlichen Emulsionen, beispielsweise Eselsmilch, Ölen und Wachsen, beispielsweise dem Bürzeldrüsenöl von Wasservögeln, diese Aufgabe zu lösen. Diese Versuche haben sich jedoch als nicht erfolgreich erwiesen. Dabei sind sowohl die Verfügbarkeiten als auch die Stabilitäten, beispielsweise gegen mikrobiellen Befall, als auch die Abscheidung erheblich zu hoher Mengen an Ölen und Wachsen auf den Fasern von großem Nachteil. Keine dieser natürlichen Emulsionen, Öle und Wachse hat sich daher erfolgreich durchgesetzt.

Überraschenderweise hat sich nun jedoch gezeigt, dass biomimetische Emulsionen hervorragend zur Reinigung und Pflege keratinischer Fasern geeignet sind. Unter biomimetischen Emulsionen werden Emulsionen verstanden, welche die natürlich vorkommenden Emulsionen in ihren Eigenschaften und Zusammensetzung nachahmen. Dieses Nachahmen der Eigenschaften in bezug auf die positiven Wirkungen an keratinischen Fasern, beispielsweise bessere Kämmbarkeiten, geringerer Spliß, höherer Glanz, gefestigte innere Struktur, angenehmes Gefühl und guter Griff der Fasern wird in hervorragender Weise von den erfindungsgemäßen biomimetischen Emulsionen erreicht. Im Vergleich zum Stand der Technik, den üblichen Emulsionen enthaltend Emulgatoren, Konditionierhilfsstoffe, Fettalkohole, kationischen Tensiden usw. werden deutlich bessere Effekte erreicht. Herkömmliche konditionierende Zusammensetzungen enthalten etwa 0,1 bis 10 Gew. % Fettalkohol, etwa 0,1 bis 5 Gew. % eines ethoxilierten Emulgators, etwa 0,1 bis 10 Gew. % eines kationischen Tensides sowie weitere Hilfs- und Zusatzstoffe.

Ein erster Gegenstand der vorliegenden Erfindung sind daher kosmetische Zusammensetzungen zur Behandlung keratinischer Fasern, insbesondere menschlicher Haare, enthaltend
a. mindestens 2,5 bis 25,0 Gew. % eines Öles oder Fettes, dessen zugrunde Triglyceride mindestens eine einfach oder mehrfach ungesättigte Fettsäure mit einer Kohlenstoffkettenlänge von mindestens 18 C - Atomen aufweisen,
b. mindestens 1,50 bis 60,0 Gew. % eines Öles oder Fettes, dessen zugrunde Triglyceride mindestens eine gesättigte Fettsäuren mit einer Kohlenstoffkettenlänge von mindestens 16 C - Atomen aufweisen,
c. mindestens 0,01 bis 7,5 Gew. % eines Proteinhydrolysates,
d. 0 bis 7,5 Gew. % eines Phospholipides und
e. einen kosmetischen Träger.

Während im Stand der Technik überwiegend geringe Mengen an Fettstoffen, vorzugsweise Fettalkohol, mit Hilfe von PEG - haltigen Emulgatoren stabilisiert wird, werden in der vorliegenden Erfindung überraschenderweise hohe Mengen an Ölen oder Fetten als Triglyceride ohne ethoxilierte Emulgatoren stabil emulgiert. Damit sind die erfindungsgemäßen biomimetischen Emulsionen milder, auf naturnahen, weitestgehend natürlichen Rohstoffen basierend und in der Wirkung gegenüber den herkömmlichen Emulsionen deutlich gesteigert.

Weiterhin zeichnen sich die erfindungsgemäßen Zusammensetzungen enthaltend den erfindungsgemäßen Wirkstoffkomplex durch einen deutlich verbesserten Zustand der keratinischen Fasern in Bezug auf den Feuchtehaushalt der keratinischen Fasern aus. Weiterhin führt der erfindungsgemäße Wirkstoffkomplex zu einem deutlichen Schutz der keratinischen Fasern vor Hitzeeinwirkungen, beispielsweise beim Föhnen keratinischer Fasern. Der Schutz der Oberfläche von keratinischen Fasern vor Hitzeeinwirkung ist insbesondere bei der Verwendung von Glätteisen oder Haartrocknern von großer Bedeutung. Schließlich wurde überraschender Weise festgestellt, dass die erfindungsgemäßen Zusammensetzungen zu einer deutlich verzögerten Wiederanschmutzung der keratinischen Fasern führen.

Soweit nachstehend vom Wirkstoffkomplex (A) gesprochen wird, bezieht sich diese Aussage auf die in den erfindungsgemäßen Mitteln zwingend enthaltenen Inhaltsstoffe a) bis e). Haarbehandlungsmittel im Sinne der vorliegenden Erfindung sind beispielsweise Haarshampoos, Haarkonditionierer, konditionierende Shampoos, Haarspülungen, Haarkuren, Haarpackungen, Haar-Tonics, Haarfärbeshampoos oder deren Kombinationen.

Unter Kämmbarkeit versteht sich erfindungsgemäß sowohl die Kämmbarkeit der nassen Faser, als auch die Kämmbarkeit der trockenen Faser.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Unter Restrukturierung im Sinne der Erfindung, ist eine Verringerung der durch verschiedenartigste Einflüsse entstandenen Schädigungen keratinischer Fasern zu verstehen. Hierbei spielt beispielsweise die Wiederherstellung der natürlichen Festigkeit eine wesentliche Rolle. Restrukturierte Fasern zeichnen sich durch einen verbesserten Glanz, durch einen verbesserten Griff und durch eine leichtere Kämmbarkeit aus. Zusätzlich weisen sie eine verbesserte Festigkeit und Elastizität auf. Ferner läßt sich eine erfolgreiche Restrukturierung physikalisch als Schmelzpunktserhöhung im Vergleich zur geschädigten Faser nachweisen. Je höher der Schmelzpunkt des Haares ist, desto fester ist die Struktur der Faser. Eine genaue Beschreibung der Bestimmung des Schmelzbereiches von Haaren findet sich in der DE 196 173 95 A1.

Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Als kosmetische Träger gemäß e) des Anspruches 1 eignen sich erfindungsgemäß besonders O/W - , W/O - und W/O/W - Emulsionen in Form von Cremes oder Gelen oder auch tensidhaltige schäumende Lösungen, wie beispielsweise Shampoos, Schaumaerosole oder andere Zubereitungen, die insbesondere für die Anwendung auf dem Haar geeignet sind. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Besonders bevorzugt ist Wasser.

Der Inhaltsstoff a) gemäß Anspruch 1 ist ein Öl oder Fett, dessen zugrunde liegendes Triglycerid mindestens eine einfach oder mehrfach gesättigte Fettsäure mit einer Kohlenstoffkettenlänge von mindestens 18 C - Atomen aufweist. Vorzugsweise ist das diesem Öl oder Fett zugrunde liegende Triglycerid so aufgebaut, mindestens zwei einfach oder mehrfach ungesättigte Fettsäuren mit einer Kohlenstoffkettenlänge von mindestens 18 C - Atomen aufweist. Besonders bevorzugt ist es jedoch, wenn die diesen Ölen oder Fetten zugrunde liegenden Triglyceride drei einfach oder mehrfach ungesättigte Fettsäuren mit einer Kettenlänge von mindestens 18C - Atomen aufweisen. Ein Beispiel für diese Öle und Fette mit einer Kettenlänge von mindestens 18 C - Atomen ist Teesamenöl. Beispiele mit mindestens zwei einfach oder mehrfach ungesättigten Fettsäuren mit einer Kohlenstoffkette von mindestens 18 C - Atomen sind Amaranthsamenöl, Aprikosenkernöl, Arganöl, Avocadoöl, Baumwollsaatöl, Babassuöl, Cashewnussöl, Distelöl, Erdnußöl, Haselnussöl, Macadamianussöl, Mandelöl, Marulaöl, Olivenöl, Palmöl, Reisöl, Sesamöl, Sonnenblumenöl, Traubenkernöl. Beispiele mit einfach, zweifach und mehrfach ungesättigten Fettsäuren mit einer Kohlenstoffkette von mindestens 18 C - Atomen sind Borretschsamenöl, Camelinaöl, Gänseschmalz, Granatapfelkernöl, Grapefruitsamenöl, Hanföl, Holundersamenöl, Johannisbeersamenöl, Kürbiskernöl, Lachsöl oder andere Fischöle aus fettreichen Fischen, Leinöl, Litschiöl, Maiskeimöl, Nachtkerzenöl, Rambutanöl, Rapsöl, Ricinusöl, Sanddornfruchtfleischöl, Sanddornkernöl, Sojaöl, Walnußöl, Weizenkeimöl, und Wildrosenöl. Selbstverständlich sind auch synthetische Triglyceride wie beispielsweise der Rohstoff Cegesoft ® PS6 (Cognis) verwendbar. Selbstverständlich sind alle anderen Fette und Öle, sofern sie die Erfordernisse an den Gehalt an ungesättigten Fettsäuren mit einer Kohlenstoffkettenlänge von mindestens 18 C - Atomen erfüllen, geeignete Öle und Fette.

Die Öle und Fette als Inhaltsstoff a) gemäß Anspruch 1 sind in einer Menge von 2,5 bis 25,0 Gew. %, vorzugsweise 5,0 bis 20,0 Gew. % und besonders bevorzugt von 8,0 bis 15 Gew. % enthalten.

Der Inhaltsstoff b) gemäß Anspruch 1 ist ein Öl oder Fett, dessen zugrunde liegendes Triglycerid mindestens eine gesättigte Fettsäure mit einer Kohlenstoffkettenlänge von mindestens 16 C - Atomen aufweist. Für den Fall, dass das Triglycerid eines Öles oder Fettes sowohl mindestens eine einfach, zweifach oder mehrfach ungesättigte Fettsäure mit einer Kettenlänge von mindestens 18 C - Atomen aufweist und gleichzeitig mindestens eine gesättigte Fettsäure mit einer Kettenlänge von mindestens 16 C - Atomen aufweist, ist der überwiegende Charakter des Öles oder Fettes ausschlaggebend. Der ausschlaggebende Charakter des Öles oder Fettes bestimmt sich nach der oder den Hauptkomponenten des Öles oder Fettes. Wenn der Anteil aller ungesättigten Fettsäuren in Summe mit einer Kohlenstoffkettenlänge von mindestens 18 C - Atomen mindestens 55 Gew. %, vorzugsweise 60 Gew. % und besonders bevorzugt 65 Gew. % und höchst bevorzugt 70 Gew. % beträgt, dann liegt ein Öl oder Fett gemäß der Definition a) des Anspruches 1 vor. Typische Beispiele für den Inhaltsstoff b) sind Kakaobutter, Rindertalg, Schweineschmalz, Lammtalg oder Butterfett. Selbstverständlich sind auch synthetische Triglyceride, welche beispielsweise aus Triglyceriden mit ungesättigten Fettsäuren durch Hydrierung enthalten werden können, sofern die Fettsäuren eine Kohlenstoffkettenlänge von mindestens 16 C - Atomen aufweisen, zu den Inhaltsstoffen gemäß b) des Anspruches 1 zu rechnen. Beispiele für den Inhaltsstoff b) sind vollständig hydriertes Ricinussöl sowie andere nahezu vollständig hydrierte Fette und Öle mit einer Kohlenstoffkettenlänge von mindestens 16 C - Atomen der zugrunde liegenden Fettsäure. Typische Beispiele für synthetische Öle und Fette sind Monomuls® 60-35 C (Cognis), Monomuls® 90-35 (Cognis), Cutina® HR (Cognis) sowie die Cegesoft® HF - Typen (Cognis). Bevorzugt ist es, wenn der Anteil an gesättigten Fettsäuren mindestens 40 Gew. %, besonders bevorzugt mindestens 50 Gew. %, ganz besonders bevorzugt mindestens 60 Gew. % und höchst bevorzugt mindestens 75 Gew. % beträgt.

Die Öle und Fette als Inhaltsstoff b) gemäß Anspruch 1 sind in einer Menge von 1,5 bis 60,0 Gew. %, vorzugsweise 3,0 bis 50,0 Gew. % und besonders bevorzugt von 5,0 bis 30 Gew. % enthalten. Die Inhaltsstoffe a) und b) gemäß Anspruch 1 werden bevorzugt in einem Gewichtsverhältnis a) zu b) von etwa 0,5 : 1 bis 1 : 5, bevorzugt 1 : 1 bis 1 : 4 verwendet. Die Gesamtmenge an den Inhaltsstoffen a) und b) beträgt mindestens 5,0 Gew. %, vorzugsweise mindestens 8,0 Gew.%, besonders bevorzugt mindestens 15,0 Gew. % und ganz besonders bevorzugt mindestens 20,0 Gew. %.

Der Inhaltsstoff c) gemäß Anspruch 1 ist ein Proteinhydrolysat. Geeignete Proteinhydrolysate sind Proteinhydrolysate sowohl auf der Basis tierischer als auch pflanzlicher, mariner oder bakterieller Herkunft der zugrunde liegenden Proteine. Die Proteinhydrolysate können sowohl durch eine saure, basische, sauer - enzymatische, basisch - enzymatische als auch ausschließlich durch enzymatische Hydrolyse gewonnen werden. Besonders bevorzugt sind Proteinhydrolysate, welche durch enzymatische Hydrolyse gewonnen wurden. Die erfindungsgemäßen Proteinhydrolysate weisen keinerlei enzymatische Aktivität mehr auf. Die erfindungsgemäßen Proteinhydrolysate weisen ein Molekulargewicht zwischen 500 Da und 5.000.000 Da auf, bevorzugt mindestens 1000 Da bis 2.000.000 Da, bevorzugter mindestens 10.000 Da bis 1.000.000 Da, besonders bevorzugt mindestens 15.000 Da bis 500.000 Da und höchst bevorzugt minstens 15.000 Da bis 100.000 Da auf. Beispiele für erfindungsgemäße Proteinhydrolysate sind Proteinhydrolysate auf der Basis von Collagen, Gelatine, Albumin, Eiprotein, Lupinenprotein, Weizenprotein, Kartoffelprotein, Rapsprotein, Sojaprotein sowie Milchproteinen. Bevorzugte Proteinhydrolysate sind Proteinhydrolysate auf der Basis von Eiprotein, Collagen, Gelatine, Sojaprotein, Albumin und Milch. Besonders bevorzugt sind Proteinhydrolysate auf der Basis von Milch. Ein Milchprotein ist auch das Casein. Mit besonderem Vorzug sind die Milchproteinhydrolysate aus Milchproteinen gewonnen, bei welchen das Casein zuvor durch Ausfällen abgetrennt wurde, so dass diese Milchproteine bei der Herstellung der Hydrolysate bis auf geringe Verunreinigungen frei von Casein sind. Übliche Milchproteine enthalten häufig Lactose. Ein mit besonderem Vorzug verwendbares Milchproteinhydrolysat liegt in dem Rohstoff Tego® Emulprot® vor.

Die Proteinhydrolysate sind in der erfindungsgemäßen Zusammensetzung in einer Menge von 0,01 bis 7,5 Gew. %, bevorzugt 0,05 bis 5,0 Gew. %, besonders bevorzugt 0,1 bis 2,0 Gew. % und höchst bevorzugt von 0,1 bis 1,5 Gew. % enthalten. Die Summe der Inhaltsstoffe aus a) und b) ist mindestens um das 10 fache größer, bevorzugt mindestens um das 20 fache, besonders bevorzugt um das 50 fache größer als die Menge an Proteinhydrolysat.

Der Inhaltsstoff d) gemäß Anspruch 1 ist ein Phospholipid. Als Phospholipid sind sowohl natürliche als auch synthetische Phospholipide verwendbar. Bevorzugt sind Phospolipide auf natürlicher Basis. Als Phospholipide sind insbesondere Verbindungen der Formel (Phosphol-1) verwendbar.

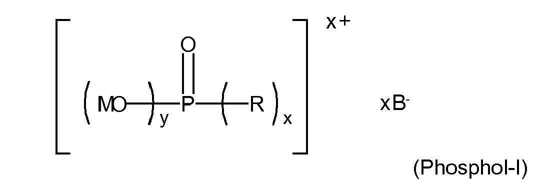

In der Formel (Phosphol-1) steht y für eine ganze Zahl von 0 bis 2, x für eine ganze Zahl von 1 bis 3 mit der Maßgabe, daß die Summe aus x und y gleich 3 ist.

In den Phospolipiden der Formel (Phospol-1) steht ferner M für Wasserstoff, ein Äquivalent eines Alkali- oder Erdalkalimetallkations, ein Ammoniumkation oder einen Alkylrest mit 1 bis 4 Kohlenstoffatomen, der gegebenenfalls mit einer oder mehreren Hydroxygruppe(n) substituiert ist. Besonders bevorzugt sind Verbindungen, bei denen M für ein Natriumkation steht.

Weiterhin steht B in der Formel (Phospol-1) der erfindungsgemäß einzusetzenden Phospolipide für ein Äquivalent eines physiologisch verträglichen Anions. Als Anion eignen sich z.B. Chlorid, Bromid, Jodid, Sulfat, Perchlorat, Tetrafluorborat, Tetraphenylborat und Tetrachlorozinkat. Bevorzugt ist das Chloridion.

R steht in der Formel (Phospol-1) für einen Rest der Formel (11), 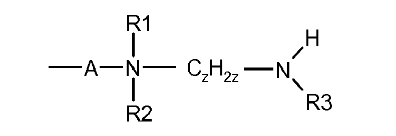 in der z für eine ganze Zahl von 1 bis 4, insbesondere für 3, steht und R¹ und R² unabhängig voneinander für einen C₁- bis C₄-Alkylrest stehen, der gegebenenfalls mit einer oder mehreren Hydroxygruppe(n) oder einer Acylgruppe substituiert ist. A steht erfindungsgemäß für eine der Einheiten -O-CH₂-CH₂-CH₂-, -O-CH₂-CH₂- oder -O-CH₂-CHOH-CH₂-, wobei die Einheit -O-CH₂-CHOH-CH₂- besonders bevorzugt ist. Der Rest R³ steht für
(a) einen verzweigten oder unverzweigten, gesättigten C₈- bis C₁₈-Acylrest oder
(b) einen verzweigten oder unverzweigten, einfach oder mehrfach ungesättigten C₈- bis C₁₈-Acylrest.

Besonders bevorzugte gesättigte Reste R³ sind der Rest der Stearinsäure sowie die Reste der Mischung der Fettsäuren, die man aus Kokosöl gewinnt.

Ein besonders bevorzugter ungesättigter Rest R³ ist der Rest der Linolsäure.

Beispiele für die als Substituenten in den erfindungsgemäßen Verbindungen genannten C₁-bis C₄-Alkylgruppen sind die Gruppen Methyl, Ethyl, Propyl, Isopropyl und Butyl. Ethyl- und Methylgruppen sind bevorzugte Alkylgruppen. Ganz besonders bevorzugt sind Methylgruppen. Ganz besonders bevorzugte Phospholipide der Formel (Phosphol-1) sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV⁰ vertrieben.

Weiterhin werden die Glycero-Phospolipide, die beispielsweise als Lecithine oder Phosphatidylcholine beispielsweise aus Eidotter oder Pflanzensamen, insbesondere Sojabohnen, gewonnen werden, als erfindungegemäße Phospholipide verwendet. Phospholipide sind insbesondere Phosphoglyceride.

Generell sind alle Phosphoglyceride geeignet. Mit Vorzug werden die im folgenden näher beschriebenen Verbindungen verwendet.Erfindungsgemäß besonders geeignete Glycero-Phospholipide werden aus Sojabohnen erhalten. Hierunter sind die Phosphatidylcholine, Phosphatidylethanolamine, Phosphatidylserine und Phosphatidylinosite sowie Mischungen dieser Substanzen besonders bevorzugt.

Die besonders bevorzugten Phosphatidylcholine weisen die Formel (Phosphol-II) auf,

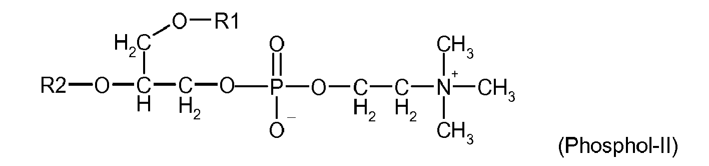

in der die Reste R¹ und R² jeweils unabhängig voneinander eine Acylgruppe aus Fettsäuren mit einer Kohlenstoffanzahl von 8 - 30 C-Atomen, bevorzugt 10 - 24 und besonders bevorzugt 12 - 22 C-Atomen, darstellen. Die Fettsäurereste können sowohl gesättigt als auch einfach oder mehrfach ungesättigt sein. Bevorzugt sind die gesättigten Acylreste von C₁₂ - C₂₂-Fettsäuren. Besonders bevorzugt sind die Acylreste der Myristinsäure, Palmitinsäure, Stearinsäure, Arachinsäure und Behensäure.

Die erfindungsgemäß bevorzugten Phosphatidylethanolamine sind solche der Formel (Phosphol-IIa) bzw. (Phosphol-Ilb), 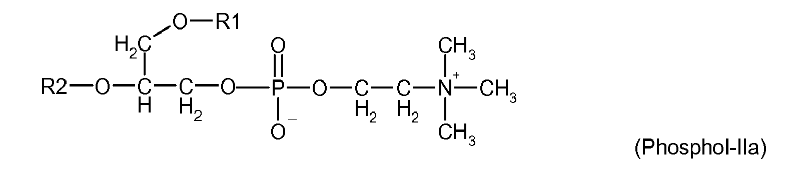 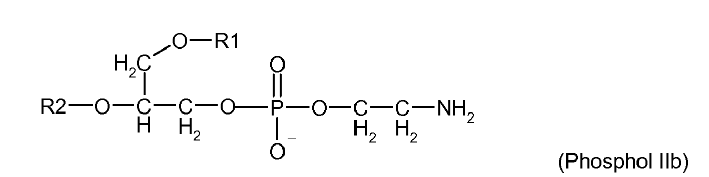 in denen die Reste R¹ und R² dieselbe Bedeutung haben wie für Formel (Phosphol-III) dargestellt. Besonders bevorzugt sind Phosphatidylethanolamine, bei denen R¹ und R² unabhängig voneinander gesättigte Acylreste von Fettsäuren mit 16 oder 18 Kohlenstoffatomen, insbesondere einen Palmitoyl- oder Stearoyl-Rest, darstellen.

Die erfindungsgemäß bevorzugten Phosphatidylserine sind solche der Strukturformel (IIIa) bzw. (lllb), 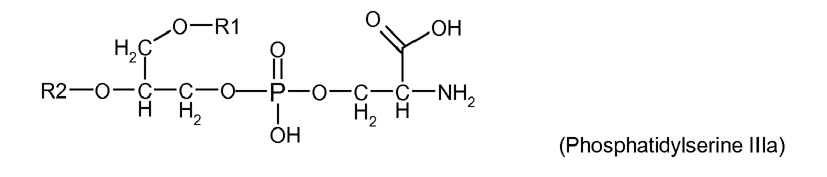 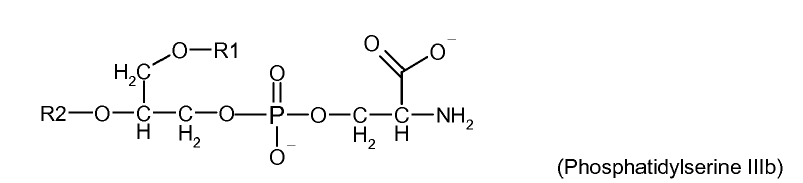 in denen R¹ und R² dieselbe Bedeutung haben wie für Formel (Phosphol-11) dargestellt. Besonders bevorzugt sind Phosphatidylserine, bei denen R¹ und R² unabhängig voneinander gesättigte Acylreste von Fettsäuren mit 16 oder 18 Kohlenstoffatomen, insbesondere einen Palmitoyl- oder Stearoyl-Rest, darstellen.

Die erfindungsgemäß bevorzugten Phosphatidylinosite weisen die Strukturformel (IVa) bzw. (IVb) auf, 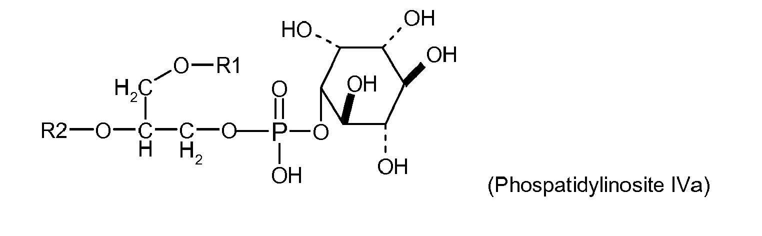 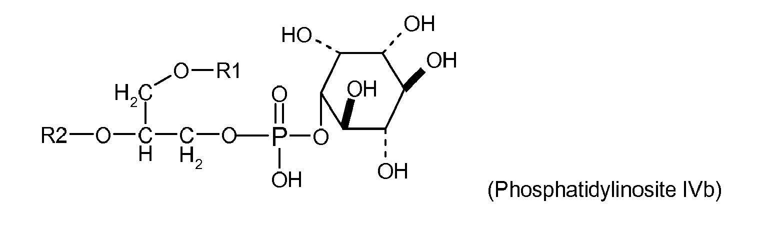 bei der die Reste R¹ und R² dieselbe Bedeutung haben wie für Formel (Phosphol-II) dargelegt. Für R¹ sind Acylreste aus Palmitinsäure, Stearinsäure und Arachinsäure bevorzugt; besonders bevorzugt ist ein Stearinsäureacylrest. R² stellt in besonders bevorzugter Weise einen linearen gesättigten C₂₀-Fettsäureacylrest (Arachoylrest) dar.

In einer besonders bevorzugten Ausführungsform sind die erfindungsgemäßen Öl-in-Wasser-Emulsionen mit einer Mischung aus gehärteten Glycero-Phospholipiden stabilisiert, die im wesentlichen der Zusammensetzung von hydriertem natürlichem Sojalecithin entspricht.

Die erfindungsgemäß verwendeten Glycero-Phospholipide weisen eine Jodzahl von maximal 10, bevorzugt von maximal 5 auf.

Erfindungsgemäß ist es auch möglich, eine Mischung aus mehreren Phospholipiden einzusetzen.

Ein erfindungsgemäß bevorzugtes Phospholipid ist unter der Bezeichnung Emulmetik® 100 (Cognis) im Handel verfügbar. Die erfindungsgemäßen Phospolipide sind in den Mitteln in Konzentrationen von 0 Gew.% bis zu 7,5 Gew.%, vorzugsweise von 0,01 Gew.% bis zu 5 Gew.%, ganz besonders bevorzugt in Mengen von 0,01 Gew.% bis zu 3 Gew.% und höchst bevorzugt inMengen von 0,1 bis zu 1,5 Gew.% enthalten.

Durch die zusätzliche Verwendung von kationischen Polymeren, bevorzugt kationischen Polymeren, welche auf einer natürlichen Rohstoffbasis hergestellt werden oder welche bereits von Haus aus kationische Biopolymere sind, beispielsweise Chitosan, wird die erfindungsgemäße Wirkstoffkombination gemäß Anspruch 1 in ihrer Wirkung weiter optimiert.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (P-3) G-O-B-N+RₐR_{b}R_{c} X⁻

G ist ein Anhydroglucoserest, beispielsweise Stärke- oder Celluloseanhydroglucose;

B ist eine divalente Verbindungsgruppe, beispielsweise Alkylen, Oxyalkylen, Polyoxyalkylen oder Hydroxyalkylen;

Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;

X- ist ein übliches Gegenanion und ist vorzugsweise Chlorid.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen.

Geeignete kationische Guarderivate werden unter der Handelsbezeichnung Jaguar vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance vertrieben. Weitere kationische Guarderivate werden von der Fa. Cognis unter der Bezeichnung Cosmedia^{®} vermarktet. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein weiteres besonders geeignetes kationisches natürliches Polymer stellen Chitosane dar. Neben dem Chitosan selbst kommen auch quaternierte, alkylierte und/oder hydroxyalkylierte Derivate, gegebenenfalls auch in mikrokristalliner Form in Betracht. Der Einsatz kann auch in Form wäßriger Gele mit einem Feststoffgehalt im Bereich von 1 bis 5 Gew.-% erfolgen.

Ein geeignetes Chitosan wird beispielsweise von der Firma Kyowa Oil& Fat, Japan, unter dem Handelsnamen Flonac^{®} vertrieben. Ein weiteres Chitosansalz wird unter der Bezeichnung Kytamer^{®} PC von der Firma Amerchol vertrieben. Als Chitosanderivate kommen quaternierte, alkylierte oder hydroxyalkylierte Derivate, beispielsweise Hydroxyethyl- oder Hydroxybutylchitosan in Betracht. Weitere Chitosanderivate sind unter den Handelsbezeichnungen Hydageno CMF, Hydagen^{®} HCMF und Chitolam^{®} NB/101 im Handel frei verfügbar.

Erfindungsgemäß bevorzugte kationische Polymere sind kationische Cellulose-Derivate und Chitosan und dessen Derivate, insbesondere die Handelsprodukte Polymero^{®} JR 400, Hydagen^{®} HCMF und Kytamer PC, kationische Guar-Derivate, kationische Honig-Derivate, insbesondere das Handelsprodukt Honeyquat^{®} 50.

Diese kationischen Polymere auf natürlicher Basis sind in den Zusammensetzungen in einer Menge von 0,01 bis 5 Gew.%, vorzugsweise 0,01 bis 3,0 Gew.% und besonders bevorzugt in einer Menge von 0,05 bis 3,0 Gew.% enthalten.

Ganz besonders bevorzugte erfindungsgemäße Zusammensetzungen gemäß Anspruch 1 enthalten weiterhin mindenstens ein Kohlenhydrat, einen Zuckeralkohol oder Zucker. Hierbei sind insbesondere Monosaccharide, Disaccharide, Trisaccharide und Oligosaccharide, Aminodesoxyzucker, Desoxyzucker Thiozucker vorteilhaft. Ganz besonders bevorzugt sind hierunter Monosaccharide mit 3 bis 8 C - Atomen, wie beispielsweise Triosen, Tetrosen, Pentosen, Hexosen, Heptosen und Octosen. Weiterhin sind höchst bevorzugt Oligosaccharide mit bis zu 50 Monomereinheiten. Beispielhaft erwähnt seien Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Trehalose, Lactose, Maltose, Cellobiose, Melibiose, Gestiobiose, Rutinose, Raffinose sowie Cellotriose. Bevorzugte Kohlenhydrate sind Sorbit, Inosit, Mannit, Threit, Erythreit, Erythrose, Threose, Arabinose, Ribose, Xylose, Glucose, Galactose, Mannose, Allose, Fructose, Sorbose, Desoxyribose, Glucosamin, Galaktosamin, Saccharose, Trehalose, Maltose und Cellobiose. Besonders bevorzugt werden Glucose, Galactose, Mannose, Fructose, Desoxyribose, Glucosamin, Saccharose, Maltose und Cellobiose verwendet. Ganz besonders bevorzugt ist jedoch die Verwendung von Glucose, Galactose, Lactose, Mannose, Fructose, Saccharose, Maltose oder Cellobiose

Unabhängig vom Typ des eingesetzten Kohlenhydrates sind erfindungsgemäße Zusammensetzungen bevorzugt, die, bezogen auf das Gewicht der Zusammensetzung, 0,01 bis 5 Gew.%, vorzugsweise 0,05 bis 4 Gew.%, besonders bevorzugt 0,05 bis 3,5 Gew.% und insbesondere 0,1 bis 2,5 Gew.% Kohlenhydrat enthalten.

Bevorzugte erfindungsgemäße Zusammensetzungen gemäß Anspruch 1 enthalten weiterhin Vitamine, Provitamine oder Vitaminvorstufen in den Zusammensetzungen enthalten.

Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05- 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Besonders bevorzugt sind aus den Vitaminen der B - Reihe die Vitamine der B3 - Reihe sowie der B5 - Reihe.

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere a-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Vitamin B, und Biotin sind ganz besonders bevorzugt.

Die Einstellung der Viskosität der erfindungsgemäßen Zusammensetzungen kann mit allen für diesen Zweck bekannten Rohstoffen erfolgen. Es ist jedoch erfindungsgemäß bevorzugt für die Einstellung der Viskosität insbesondere nichtionische Polymere auf natürlicher Basis zu wählen. Dies sind insbesondere Xanthangumme, beispielsweise Keltrol® - Typen, Guargumme, Gelatine, Pectine, Stärken, beispielsweise aus Kartoffeln, Reis, oder Mais. Insbesondere die Stärke wird in einer vorverkleisterten Form verwendet. Derartige Produkte sind von den Fimen Avebe, Hercules, National Starch und anderen im Handel verfügbar. Die nichtionischen Polymere auf natürlicher Basis sind in den erfindungsgemäßen Zusammensetzungen in einer Menge von 0,01 bis 10 Gew. %, bevorzugt 0,01 bis 7,5 Gew. %, besonders bevorzugt 0,05 bis 5,0 Gew. % jeweils bezogen auf das Gewicht der gesamten Zusammensetzung enthalten.

Weitere Inhaltsstoffe der Zusammensetzungen gemäß Anspruch 1 können alle weiteren in kosmetischen Mitteln üblichen Inhaltsstoffe sein. Der Fachmann wird diese je nach dem Zweck des Mittels gezielt auswählen. Beispielsweise werden reinigende Zusammensetzungen zusätzlich anionische, amphotäre, nicht-ionische oder kationische Tenside enthalten. Im Folgenden werden einige ganz besonders bevorzugte weitere Inhaltsstoffe beipsielhaft beschrieben. Um dem biomimetischen Charakter der Zusammensetzungen Rechnung zu tragen, sind als weitere Inhaltsstoffe insbesondere solche bevorzugt, die möglichst naturnah, naturidentisch oder auf einer natürlichen Rohstoffbasis beruhen.

Die erste Gruppe weiterer Inhaltsstoffe sind Pflanzenextrakte.Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea angustifolia DC, Echinacea paradoxa (Norton), Echinacea simulata, E. atrorubens, E. tennesiensis, Echinacea strigosa (Mc Gregor), Echinacea laevigata, Echinacea purpurea (L.) Moench und Echinacea pallida (Nutt), aller Arten von Wein sowie Perikarp von Litchie chinensis bevorzugt.

Die Pflanzenextrakte können erfindungsgemäß sowohl in reiner als auch in verdünnter Form eingesetzt werden. Bezogen auf den Gehalt des jeweiligen Pflanzenextraktes werden Pflanzenextrakte in solchen Mengen verwendet, dass in den erfindungsgemäßen Zusammensetzungen Wirkstoffgehalte des jeweiligen Extraktes in einer Menge von 0,001 bis 7,5 Gew. %, bevorzugt 0,01 bis 5,0 Gew. % und besonders bevorzugt von 0,01 bis 3,0 Gew. % enthalten sind.

Erfindungsgemäß können selbstverständlich neben den zwingend erforderlichen Proteinhydrolysaten gemäß Anspruch 1, Punkt c) weitere Proteinhydrolysate zur Erzielung von Pflegeeffekten verwendet werden. Hierzu können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Moringa-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy (Croda), Hydrolupin^{®} (Croda), Hydrosesame^{®} (Croda), Hydrotritium^{®} (Croda), Crotein^{®} (Croda) und Puricare^{®} LS 9658 von der Fa. Laboratoires Särobiologiques erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Die Proteinhydrolysate (P) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Eine besonders bevorzugte Gruppe von Inhaltsstoffen in den erfindungsgemäßen kosmetischen Zusammensetzungen sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. In einer besonders bevorzugten Ausführungsform der Erfindung wird als Wirkstoff L-Carnitintartrat eingesetzt. Erfindungsgemäß besonders bevorzugt sind Mittel, die - bezogen auf ihr Gewicht - 0,00001 bis 10,0 Gew.-%, vorzugsweise 0,0001 bis 5,0 Gew.-% und insbesondere 0,001 bis 3 Gew.-% der genannten Betaine enthält.

Ein besonders wesentlicher Inhaltsstoff ist Taurin und/oder ein Derivat des Taurines. Unter Taurin wird ausschließlich 2-Aminoethansulfonsäure und unter einem Derivat werden die explizit genannten Derivate des Taurines verstanden. Unter den Derivaten des Taurines werden N-Monomethyltaurin, N, N-Dimethyltaurin, Taurinlysylat, Taurintartrat, Taurinornithat, Lysyltaurin und Ornithyltaurin verstanden. Weitere Taurinderivate im Sinne der vorliegenden Erfindung sind die Taurocholsäure und Hypotaurin.

Besonders bevorzugt sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. In einer weiteren erfindungsgemäß bevorzugten Ausführungsform enthalten die erfindungsgemäßen Zusammensetzungen Biochinone. In den erfindungsgemäßen Mitteln sind unter geeigneten Biochinonen ein oder mehrere Ubichinon(e) und/oder Plastochinon(e) zu verstehen. Die erfindungsgemäß bevorzugten Ubichinone weisen die folgende Formel auf: 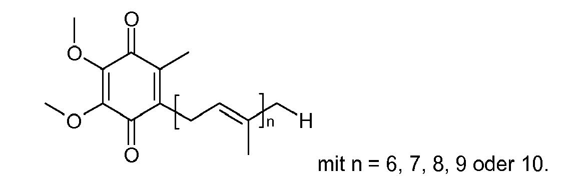 Das Coenzym Q-10 ist hierbei am bevorzugtesten.

Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Erfindungsgemäß bevorzugte kosmetische Mittel sind dadurch gekennzeichnet, daß sie mindestens eines der folgenden Purine enthalten: Purin, Adenin, Guanin, Harnsäure, Hypoxanthin, 6-Purinthiol, 6-Thioguanin, Xanthin, Coffein, Theobromin oder Theophyllin.

Je nach gewünschtem Anwendungszweck der kosmetischen Mittel kann dabei die Art und Menge des Purinderivates variieren. In haarkosmetischen Formulierungen hat sich insbesondere Coffein bewährt, das beispielsweise in Shampoos, Conditionern, Haarwässern und/oder Lotionen vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf die Zusammensetzung) eingesetzt werden kann.

Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetio9 SN), 2-Ethylhexylpalmitat (Cegesoft^{®} 24), Stearinsäure-2-ethylhexylester (Cetiol^{®} 868), Cetyloleat, Glycerintricaprylat, Kokosfettaikohoi-caprinat/-capryiat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V). Selbstverständlich können die Esteröle auch mit Ethylenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein. Bevorzugt ist erfindungsgemäß jedoch, wenn zunächst der Fettalkohol alkoxyliert wurde und anschließend mit Fettsäure verestert wurde. In der Formel (D4-11) sind allgemein diese Verbindungen dargestellt. 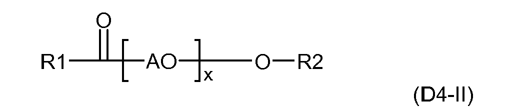 R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetio9 CC),
- Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet. Selbstverständlich ist es erfindungsgemäß auch möglich mehrere Esteröle gleichzeitig zu verwenden.

In vielen Fällen enthalten die Zusammensetzungen mindestens eine oberflächenaktive Substanz, wobei prinzipiell sowohl anionische als auch zwitterionische, ampholytische, nichtionische und kationische oberflächenaktive Substanzen geeignet sind. Die Auswahl der oberflächenaktiven Substanzen richtet sich einerseits nach der Art des Mittels. Andererseits werden die Tenside ganz besonders bevozugt so ausgewählt, dass sie weitestgehend auf einer natürlichen Basis beruhen. Im Falle eines Shampoos wird insbesondere mindestens ein Tensid aus der Gruppe der anionischen, der zwitterionischen oder nichtionischen oberflächenaktiven Substanzen gewählt. Bevorzugt ist hierbei, dass mindestens eine anionische und mindestens eine zwitterionische oberflächenaktive Substanz gewählt wird. Besonders bevorzugt werden diese oberflächenaktiven Substanzen dabei aus der Gruppe der besonders milden oberflächenaktiven Substanzen gewählt. Als anionische Tenside (Tanion) eignen sich in erfindungsgemäßen Zubereitungen alle für die Verwendung am menschlichen Körper geeigneten anionischen oberflächenaktiven Stoffe. Typische Beispiele besonders bevorzugter anionischer Tenside sind:
- lineare und verzweigte Fettsäuren mit 8 bis 30 C-Atomen (Seifen),
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe,
- Alkylsulfate,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, R¹ (OCH₂CH₂)ₙ-O-(PO-OX)-OR²,
   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 0 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- Monoglyceridsulfate und Monoglyceridethersulfate der Formel R⁸OC-(OCH₂CH₂)x-OCH₂-[CHO(CH₂CH₂O)yH]-CH₂O(CH₂CH₂O),-SO₃X,
   in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der R⁸CO für einen linearen Acylrest mit 8 bis 18 Kohlenstoffatomen steht,
- Amidethercarbonsäuren, R¹-CO-NR²-CH₂CH₂O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R^{s}R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypö erhältlich.
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht,
- Kondensationsprodukte aus einem wasserlöslichen Salz eines wasserlöslichen Eiweißhydrolysats mit einer C8 - C30 - Fettsäure. Solche Produkte sind unter dem Warenzeichen Lamepon^{®}, Maypon^{®}, Gluadin^{®}, Hostapon^{®} KCG oder Amisoft^{®} seit langem im Handel erhältlich.
- Alkyl- und/oder Alkenyl-Oligoglykosidcarboxylate, -sulfate, -phosphate und/oder -isethionate
   und
- Acyllactylate.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugte ampholytische Tenside sind das N-Kokosalkylaminopropionat, das Kokosacylaminoethylaminopropionat und das C₁₂ - C₁₈ Acylsarcosin.

Nichtionische Tenside (Tnio) sind beispielsweise
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydageno HSP (Cognis) oder Sovermol^{®} - Typen (Cognis),
- alkoxilierte Triglyceride,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Polyglycerine und Polyglycerinderivate wie beispielsweise Polyglycerinpoly-12-hydroxystearat (Handelsprodukt Dehymuls^{®} PGPH),
- Zuckertenside vom Typ der Alkyl- und Alkenyloligoglykoside und
- Zuckertenside vom Typ der Fettsäure-N-alkylpolyhydroxyalkylamide.

Als kationische Tenside können erfindungsgemäß alle dem Fachmann bekannten und übliche kationischen Tenside verwendet werden. Es ist jedoch bevorzugt auf kationische Tenside nach Möglichkeit zu verzichten. Ist die nicht möglich, so werden die folgenden kationischen Tenside bevorzugt eingesetzt. Hierzu zählen:
- quartäre Imidazolinverbindung. Die im Folgenden dargestellte Formel Quimi-1 zeigt die Struktur dieser Verbindungen. 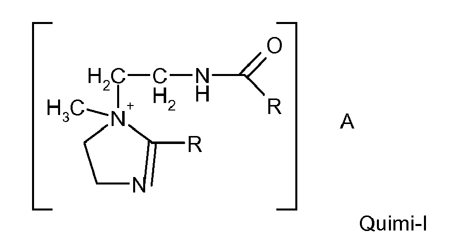 Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel I enthalten für R jeweils den gleichen Kohlenwasserstoffrest. Die Kettenlänge der Reste R beträgt bevorzugt 12 bis 21 Kohlenstoffatome. Besonders erfindungsgemäße Beispiele sind beispielsweise unter den INCII
- Bezeichnungen Quaternium-27, Quaternium-72, Quaternium-83 und Quaternium-91 erhältlich.
- kationische Tenside gemäß der Formel (Tkat-2),

RCO-X-N⁺R¹R²R³ A⁻ (Tkat-2)

R steht hierin für einen substituierten oder unsubstituierten, verzweigten oder geradkettigen Alkyl- oder Alkenylrest mit 11 bis 35 Kohlenstoffatomen in der Kette, X steht für - 0 - oder - NR⁵ -,
R¹ steht für eine Alkylengruppe mit 2 bis 6 C - Atomen, welche nicht substituiert oder substituiert sein kann, wobei im Falle einer Substitution die Substitution mit einer -OH - oder - NH- Gruppe bevorzugt ist,
R², R³ jeweils unabhängig voneinander stehen für eine Alkyl oder Hydroxyalkylgruppe mit 1 bis zu 6 C - Atomen in der Kette, wobei die Kette geradlinig oder verzweigt sein kann. R5 steht für Wasserstoff oder einen C1 bis C6 geradkettigen oder verzweigten, Alkyl- oder Alkenylrest, welcher auch durch eine Hydroxygruppe substituiert sein kann. Innerhalb dieser Strukturklasse werden bevorzugt die Verbindungen einer der folgenden Strukturen verwendet:

| | | |
|---|---|---|
| CH₃(CH_{z})_{zo}CONH(CH_{z})₃ - N⁺(CH₃)₂-CH₂CH₃ | A⁻ | (Tkat-3) |
| CH₃(CH₂)₂₀CONH(CH₂ₕ - N⁺(CH₃)₂-CH₂(CHOH)CH₂OH | A⁻ | (Tkat-4) |
| CH₃(CH2)_{zo}COOCH_{z}CHOHCH_{z} - N⁺(CH₃)₃ | A⁻ | (Tkat-5) |
| CH₃(CH_{z})_{zo}CONH(CH_{z})₃ - N⁺(CH₃)₂-CH₂CH₂OH | A⁻ | (Tkat-6) |

Beispiele für derartige Handelsprodukte sind Schercoquat BAS, Lexquat AMG-BEO,

Akypoquat 131 oder Incroquat Behenyl HE.
- Esterquats gemäß der Formel (Tkat1-2) verwendet werden. 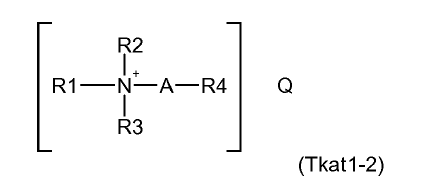 Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, daß höchstens 2 der Reste R1, R2 oder R3 für diesen Rest stehen können:
   Der Rest -(A - R4) ist mindestens 1 bis 3 mal enthalten.
   Hierin steht A für:
      1) -(CH2)n- mit n = 1 bis 20, vorzugsweise n = 1 bis 10 und besonders bevorzugt n = 1 - 5, oder
      2) -(CH2-CHR5-0)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl, und
   R4 steht für:
      1) R6-0-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
      2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, und
- Q steht für ein physiologisch verträgliches organisches oder anorganisches Anion. Solche Produkte werden beispielsweise unter den Warenzeichen Rewoquat, Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat® WE18, Rewoquat^{®} WE38 DPG und Stepantex^{®} VS 90 sind Beispiele für solche Esterquats.

Weitere erfindungsgemäß besonders bevorzugte Verbindungen der Formel (Tkat1-2) zählen zur Formel (Tkat1-2.1), den kationischen Betainestern. 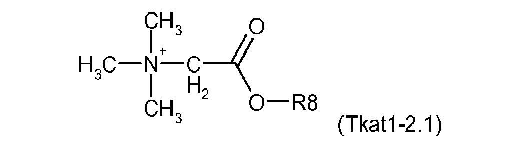 R8 entspricht in seiner Bedeutung R7.
- Monoalkyltrimethylammoniumsalze mit einer Kettenlänge des Alkylrestes von 16 bis 24 Kohlenstoffatomen entsprechend der Formel (Tkat1-1), 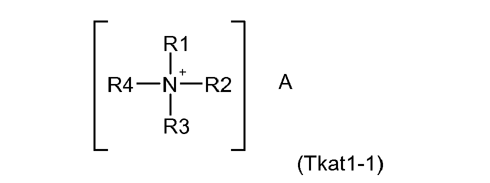 in denen R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen. Beispiele für Verbindungen der Formel (Tkat1-1) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.
- Amine und/oder kationisiertes Amine, insbesondere ein Amidoamin und/oder ein kationisiertes Amidoamin mit den folgenden Strukturformeln:

| | | | |
|---|---|---|---|
| R¹ - NH - (CH₂)ₙ - NR²R³ | (Tkat7) | | und/oder |
| R¹ - NH - (CH₂)ₙ - NR²R³R⁴ | (Tkat8) | | |

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und R2, R3 und R4 jeweils unabhängig voneinander Wasserstoff oder ein Alkylrest mit 1 bis 4 C-Atomen, welcher gleich oder verschieden, gesättigt oder ungesättigt sein kann, und X- ein Anion und n eine ganze Zahl zwischen 1 und 10 bedeuten.

Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen-oder Candellilawachse in Betracht.Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5. Weiterhin bevorzugt sind Amidoamine und/oder quaternisierte Amidoamine der allgemeinen Formeln (Tkat7) und/oder (Tkat8), in denen das Anion X ein Halogenidion oder eine Verbindung der allgemeinen Formel RSO₃ ist, worin R die Bedeutung von gesättigtem oder ungesättigtem Alkylresten mit 1 bis 4 Kohlenstoffatomen hat. Der Alkylrest mit 1 bis 4 Kohlenstoffatomen von R2, R3 und R4 und/oder der Alkylrest mit 1 bis 4 Kohlenstoffatomen von RSO₃ in der allgemeinen Formel (Tkat7) und/oder (Tkat8) können mindestens eine Hydroxylgruppe enthalten. Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine. Als erfindungsgemäß zu verwendende Amidoamine, welche gegebenenfalls quaternisiert sein können, kommen beispielsweise in Betracht als Amidoamine: Witcamine® 100 (Witco, INCI-Bezeichnung: Cocamidopropyl Dimethylamine), Incromine® BB (Croda, INCI-Bezeichnung: Behenamidopropyl Dimethylamine), Mackine® 401 (Mclntyre, INCI-Bezeichnung: Isostearylamidopropyl Dimethylamine) und andere Mackine-Typen, Adogen® S18V (Witco, INCI-Bezeichnung: Stearylamidopropyl Dimethylamine), und als permanent kationische Aminoamine: Rewoquat® RTM 50 (Witco Surfactants GmbH, INCI-Bezeichnung: Ricinoleamidopropyltrimonium Methosulfate), Empigen® CSC (Albright&Wilson, INCI-Bezeichnung: Cocamidopropyltrimonium Chlorid), Swanol® Lanoquat DES-50 (Nikko, INCI-Bezeichnung: Quatemium-33), Rewoquat® UTM 50 (Witco Surfactants GmbH, Undecyleneamidopropyltrimonium Methosulfate).

Das Anion der aller zuvor beschriebenen kationischen Verbindungen ist ausgewählt aus den physiologisch verträglichen Anionen. Beispielhaft hierfür seien die Halogenidionen, Fluorid, Chlorid, Bromid, Sulfat der allgemeinen Formel RS0₃ , worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Bevorzugt werden kationische Imidazoline, Esterquats, kationische Tenside gemäß der Formel (Tkat-2) sowie Amine und/oder kationisiertes Amine, insbesondere Amidoamine und/oder kationisiertes Amidoamine verwendet.

Die zuvor genannten kationischen Tenside können einzeln oder in beliebigen Kombinationen miteinander verwendet werden, wobei Mengen zwischen 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew. % jeweils bezogen auf die Gesamtzusammensetzung des jeweiligen Mittels erhalten.

Die Tenside (T) werden in einer Gesamtmenge der Tenside in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Als Fettsäuren (Fatac) können eingesetzt werden lineare und/oder verzweigte, gesättigte und/oder ungesättigte Fettsäuren mit 6-30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und isopaimitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew. %, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilano käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 1 - 20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Ferner können die kosmetischen Mittel weitere Wirk-, Hilfs- und Zusatzstoffe enthalten, wie beispielsweise
- UV - Lichtschutzfilter mit einer Wirkung im Bereich des UV-A, UV-B und UV-C Lichtes sowohl als wasserlösliche als auch öllösliche Filter,
- Weitere kationische, amphotere, anionische oder nichtionische Polymere auf synthetischer Basis,
- Silikone wie beispielsweise Cyclomethicone, Dimethicone, Dimethiconole, Dimethiconcopolyole,
- Farbstoffvorprodukte vom Kuppler- und Entwicklertyp,
- Direktziehende Farbstoffe,
- Strukturanten wie Maleinsäure und Milchsäure,
- Quellmittel wie Harnstoff, Allantoin, Carbonate oder Hydantoin,
- Dimethylisosorbid und Cyclodextrine,
- Farbstoffe zum Anfärben des Mittels,
- Antischuppenwirkstoffe wie Piroctone Olamine, Zink Omadine und Climbazol,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂0, Dimethylether, CO₂ und Luft,
- Antioxidantien,
- Parfümöle, Duftstoffe und Riechstoffe.

Bezüglich weiterer fakultativer Komponenten sowie die eingesetzten Mengen dieser Komponenten wird ausdrücklich auf die dem Fachmann bekannten einschlägigen Handbücher verwiesen.

Wie bereits erwähnt, kommt der hohen Pflegewirkung der erfindungsgemäßen Mittel insbesondere daher Bedeutung zu, als sie auch in Gegenwart von Oxidationsmitteln - beispielsweise im Rahmen der oxidativen Haarfärbung - hervorragende Ergebnisse liefert.

Ein zweiter Erfindungsgegenstand ist daher ein Verfahren zur Haarbehandlung, in dem ein kosmetisches Mittel gemäß Anspruch 1 auf das Haar aufgetragen wird und nach einer Einwirkungszeit vom Haar gespült wird.

Die Einwirkungszeit beträgt bevorzugt wenige Sekunden bis 100 Minuten, besonders bevorzugt 1 bis 50 Minuten und ganz besonders bevorzugt 1 bis 30 Minuten.

Die folgenden Beispiele sollen den Gegenstand der vorliegenden Erfindung erläutern ohne ihn jedoch zu beschränken.

### Beispiele und Wirkungsnachweis

Alle Mengenangaben sind, soweit nicht anders vermerkt, Gewichtsteile. Die folgenden Rezepturen wurden unter Anwendung bekannter Herstellungsverfahren bereitgestellt.

### 1. Wirkungsnachweis der Restrukturierung

### Vorbehandlung

Strähnen der Fa. Kerling (1,0 g, Code ENH 7/0) wurden mit dem Blondiermittel Igora Vario Blond Plus mit 9,0 % Wasserstoffperoxid im Mischungsverhältnis 1:2 ohne vorheriges Waschen für 30 Minuten blondiert. Danach wurden die Strähnen gründlich mit Wasser von 32 °C mindestens 1 Minute ausgespült. Es folgte im nassen Zustand im unmittelbaren Anschluß an die Blondierung eine herkömmliche Dauerwellbehandlung mit 2,0 g des Produktes Natural Styling 0, Classic. Im Rahmen dieser Dauerwellbehandlung wurden die Fasern in einem ersten Schritt für 10 Minuten bei Raumtemperatur der Reduktionslösung (enthaltend 7,9 Gew.-% Thioglykolsäure) ausgesetzt, mit reinem Wasser mit einer Temperatur von 32 °C für 1 Minute unter fließendem Wasser gespült, mit einem Handtuch getrocknet und anschließend bei Raumtemperatur für 10 Minuten mit 2,0 g der zugehörigen Oxidationslösung fixiert. Die Oxidationslösung enthielt 2,6 Gew.-% Wasserstoffperoxid. Nach der oxidativen Behandlung wurden die Fasern wiederum für 1 Minute unter fließendem Wasser bei 32 °C gespült und anschließend über nacht an der Luft getrocknet.

### Nachbehandlung

Die Strähnen wurden jeweils bei einer Temperatur von 32°C 1 Minute mit fließendem Wasser gespült. Danach wurde jede Strähne 5Minuten in die entsprechenden Zusammensetzungen getaucht. Nach der Behandlung mit der jeweiligen Zusammensetzung wurde jede Haarsträhne 1 Minute mit klarem Wasser mit einer Temperatur von 32 °C 1 Minute ausgespült, an der Luft getrocknet und 16 h ruhen gelassen.

### Nachweis der haarstrukturierenden Wirkung mittels HP-DSC

Mittels einer DSC-Analyse (Perkin Elmer DSC-7) wurden die folgenden in Tabelle 1 dargestellten Schmelzpunkte ermittelt. Eine genaue Beschreibung der Methode findet sich beispielsweise in der DE 196 173 95 A1.

**Tabelle 1: Zusammensetzungen in Gew. %**

| Rohstoff | Emulsion 1 | Emulsion 2 |
|---|---|---|
| | (erfindungsgemäß) | (Stand der Technik) |
| Cegesoft® HF 52 | 10,0 | - |
| Cegesoft® HF 62 | 12,0 | - |
| Cegesoft® PS 6 | 6,0 | - |
| Emulmetik® 100 | 1,2 | - |
| Milchproteinhydrolysat | 0,4 | - |
| Lanette® O | - | 6,25 |
| Varisoft® W 575 | - | 0,75 |
| Cutina® AGS | - | 1,0 |
| Cegesoft® SBE | - | 1,5 |
| Rambutanöl | - | 0,5 |
| Quartamin® BTC 131 | - | 1,5 |
| Dehyquart® F75 | - | 0,75 |
| Milchsäure | - | 0,1 |
| Cosmedia CTH | - | 0,4 |
| Glycerin | - | 0,1 |
| Dow Corning 200, 60.000 cSt | 200, - | 1,0 |
| Konservierung, Parfüm | q.s. | q.s. |
| Wasser | Ad 100 | Ad 100 |

**Tabelle 2: Wirkungsnachweis - Ergebnisse:**

| Wirkstoff bzw. Wirkstoffkomplex | Schmelzpunkt in °C |
|---|---|
| kaltgewelltes und blondiertes Haar ohne Wirkstoff | 144 |
| Emulsion 1 (erfindungsgemäß) | 147 |
| Emulsion 2 (Stand der Technik) | 144 |

Das Signifikanzniveau der erfindungsgemäßen Emulsion gegenüber dem Wert für unbehandeltes Haar beträgt >95%.

### 2. Weitere Beispiele der Erfindung

Alle Mengenangaben sind, soweit nicht anders vermerkt, Gewichtsteile. Die folgenden Rezepturen wurden unter Anwendung bekannter Herstellungsverfahren bereitgestellt.

Haarpflegeemulsionen

| | **Emulsion 3** | **Emulsion 4** |
|---|---|---|
| Argan Oil | 22 | ---- |
| Phospholipon 50 | 0,4 | ---- |
| Milchproteinhydrolysat | 1,2 | ---- |
| GPR Rectapur | 4 | ---- |
| Panthenol | 1 | ---- |
| Glycerin | 0,2 | ---- |
| Rambutan Oil | ---- | 18 |
| Cegesoft PS 6 | ---- | 15 |
| Casein | ---- | 0,3 |
| Gluadin WK | ---- | 2,4 |
| Phosal 35 SB | ---- | 1,8 |
| Xanthan Gum | ---- | 0,3 |
| Tocopherol Acetate | ---- | 1 |
| Wasser, Konservierung, Parfüm | ad 100 | ad 100 |

## Patentansprüche

1. Kosmetische Zusammensetzung zur Behandlung keratinischer Fasern, insbesondere menschlicher Haare enthaltend
a. mindestens 2,5 bis 25,0 Gew. % eines Öles oder Fettes, dessen zugrunde Triglyceride mindestens eine einfach oder mehrfach ungesättigte Fettsäure mit einer Kohlenstoffkettenlänge von mindestens 18 C - Atomen aufweisen,
b. mindestens 1,50 bis 60,0 Gew. % eines Öles oder Fettes, dessen zugrunde Triglyceride mindestens eine gesättigte Fettsäuren mit einer Kohlenstoffkettenlänge von mindestens 16 C - Atomen aufweisen,
c. mindestens 0,01 bis 7,5 Gew. % eines Proteinhydrolysates,
d. 0 bis 7,5 Gew. % eines natürlichen Phospholipides und
e. einen kosmetischen Träger.

2. Kosmetische Zusammensetzung nach Anspruch 1, **dadurch gekennzeichnet, dass** die den Ölen oder Fetten gemäß a) zugrunde liegenden Triglyceride mindestens zwei einfach oder mehrfach ungesättigte Fettsäuren mit einer Kohlenstoffkettenlänge von mindestens 18 C -Atomen aufweisen.

3. Kosmetische Zusammensetzung nach Anspruch 1, **dadurch gekennzeichnet, dass** die den Ölen oder Fetten gemäß a) zugrunde liegenden Triglyceride drei einfach oder mehrfach ungesättigte Fettsäuren mit einer Kohlenstoffkettenlänge von mindestens 18 C - Atomen aufweisen.

4. Kosmetische Zusammensetzung nach Anspruch 1, **dadurch gekennzeichnet, dass** die den Ölen oder Fetten gemäß b) zugrunde liegenden Triglyceride mindestens zwei gesättigte Fettsäuren mit einer Kohlenstoffkettenlänge von mindestens 16 C - Atomen aufweisen.

5. Kosmetische Zusammensetzung nach Anspruch 1, **dadurch gekennzeichnet, dass** die den Ölen oder Fetten gemäß b) zugrunde liegenden Triglyceride drei gesättigte Fettsäuren mit einer Kohlenstoffkettenlänge von mindestens 16 C - Atomen aufweisen.

6. Kosmetische Zusammensetzung nach einem der Ansprüche 1 bis 5, **dadurch gekennzeichnet, dass** weiterhin mindestens ein Vitamin der B - Reihe enthalten ist.

7. Kosmetische Zusammensetzung nach einem der Ansprüche 1 oder 6, **dadurch gekennzeichnet, dass** weiterhin mindestens ein kationisches Polymer enthalten ist.

8. Kosmetische Zusammensetzungen nach einem der Ansprüche 1 bis 7, **dadurch gekennzeichnet, dass** mindestens ein Polysaccharid enthalten ist.

9. Verfahren zur Behandlung keratinischer Fasern **dadurch gekennzeichnet, dass** eine kosmetische Zusammensetzung gemäß einem der Ansprüche 1 bis 7 auf die keratinischen Fasern aufgetragen wird und nach einer Einwirkzeit von wenigen Sekunden bis hin zu 45 Minuten wieder ausgespült wird.

10. Verwendung eines Mittels gemäß einem der Ansprüche 1 bis 8 zur Restrukturierung keratinischer Fasern.
